(19) Europäisches Patentamt
European Patent Office
Office européen des brevets

(11) **EP 2 869 760 B1**

(12) **EUROPÄISCHE PATENTSCHRIFT**

(45) Veröffentlichungstag und Bekanntmachung des Hinweises auf die Patenterteilung:
**17.08.2016  Patentblatt 2016/33**

(21) Anmeldenummer: **12794668.9**

(22) Anmeldetag: **14.11.2012**

(51) Int Cl.:
*A61B 5/05* (2006.01)     *H03M 7/30* (2006.01)
*G06T 11/00* (2006.01)     *G01R 33/12* (2006.01)
*G01R 33/00* (2006.01)

(86) Internationale Anmeldenummer:
**PCT/EP2012/072550**

(87) Internationale Veröffentlichungsnummer:
**WO 2014/005658 (09.01.2014 Gazette 2014/02)**

(54) **KALIBRIERVERFAHREN FÜR EINE MPI (=MAGNETIC-PARTICLE-IMAGING)-APPARATUR**

CALIBRATION METHOD FOR AN MPI (=MAGNETIC PARTICLE IMAGING) APPARATUS

PROCÉDÉ D'ÉTALONNAGE POUR APPAREIL DE MAGNÉTOSCOPIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.07.2012   DE 102012211662**

(43) Veröffentlichungstag der Anmeldung:
**13.05.2015   Patentblatt 2015/20**

(73) Patentinhaber: **Bruker BioSpin MRI GmbH 76275 Ettlingen (DE)**

(72) Erfinder: **KNOPP, Tobias 76337 Waldbronn (DE)**

(74) Vertreter: **Kohler Schmid Möbus Patentanwälte Partnerschaftsgesellschaft mbB Ruppmannstraße 27 70565 Stuttgart (DE)**

(56) Entgegenhaltungen:
US-A1- 2011 221 438

• WEIZENECKER J ET AL: "LETTER TO THE EDITOR; Three-dimensional real-time in vivo magnetic particle imaging", PHYSICS IN MEDICINE AND BIOLOGY, INSTITUTE OF PHYSICS PUBLISHING, BRISTOL GB, Bd. 54, Nr. 5, 7. März 2009 (2009-03-07), Seiten L1-L10, XP020149861, ISSN: 0031-9155, DOI: 10.1088/0031-9155/54/5/L01 in der Anmeldung erwähnt

• HALKOLA A ET AL: "System calibration unit for magnetic particle imaging: Focus field based system function", 1. März 2012 (2012-03-01), MAGNETIC PARTICLE IMAGING, SPRINGER, PAGE(S) 27 - 31, XP009167740, ISBN: 3-642-24132-8 in der Anmeldung erwähnt das ganze Dokument

• CANDES E J ET AL: "An Introduction To Compressive Sampling", IEEE SIGNAL PROCESSING MAGAZINE, IEEE SERVICE CENTER, PISCATAWAY, NJ, US, Bd. 25, Nr. 2, 1. März 2008 (2008-03-01), Seiten 21-30, XP011225660, ISSN: 1053-5888, DOI: 10.1109/MSP.2007.914731

• KNOPP T ET AL: "Model-Based Reconstruction for Magnetic Particle Imaging", IEEE TRANSACTIONS ON MEDICAL IMAGING, IEEE SERVICE CENTER, PISCATAWAY, NJ, US, Bd. 29, Nr. 1, 1. Januar 2010 (2010-01-01) , Seiten 12-18, XP011293583, ISSN: 0278-0062 in der Anmeldung erwähnt

• GOODWILL P W ET AL: "Projection X-Space Magnetic Particle Imaging", IEEE TRANSACTIONS ON MEDICAL IMAGING, IEEE SERVICE CENTER, PISCATAWAY, NJ, US, Bd. 31, Nr. 5, 1. Mai 2012 (2012-05-01), Seiten 1076-1085, XP011491091, ISSN: 0278-0062, DOI: 10.1109/TMI.2012.2185247

EP 2 869 760 B1

**Beschreibung**

Hintergrund der Erfindung

Die Erfindung betrifft ein Kalibrierverfahren einer MPI-Apparatur zur Durchführung eines MPI-Experiments, wobei das Kalibrierverfahren M Kalibrier-MPI-Messungen mit einer Kalibrierprobe umfasst und aus diesen eine Bildrekonstruktionsmatrix erzeugt, mit der die Signalbeiträge von N Voxeln innerhalb eines Untersuchungsvolumens der MPI-Apparatur ermittelt werden.

Ein derartiges Verfahren ist beispielsweise bekannt aus den Referenzen [WGR09] oder [KSB10].

Magnetic-Particle-Imaging (abgekürzt "MPI") ist ein neues bildgebendes Verfahren, das es ermöglicht, die örtliche Verteilung magnetischer Nanopartikel zu bestimmen [WGR09]. Hierzu werden die Partikel verschiedenen statischen und dynamischen Magnetfeldern ausgesetzt und die Magnetisierungsänderungen der Partikel mittels Empfangsspulen detektiert. Zur Ortskodierung wird bei MPI ein magnetisches Gradientenfeld genutzt, das einen feldfreien Punkt (FFP) hat. Durch Verschiebung des FFPs entlang einer vordefinierten Trajektorie kann der zu untersuchende Messbereich abgetastet werden. Hierzu werden dynamische Anregungsfelder genutzt (engl. drive field).

Der Zusammenhang zwischen der zu rekonstruierenden Partikelverteilung $c(r)$ und den spektralen Koeffizienten des Messsignals $\hat{u}_k$ $k=0,...,K-1$ kann durch eine lineare Integralgleichung

$$\hat{u}_k = \int_{Objekt} c(r) s_k(r) d^3 r \qquad (1)$$

beschrieben werden. Dabei bezeichnet $s_k(r)$ die Systemfunktion, welche sowohl vom Ort r als auch von dem Frequenzindex k abhängt. Die Anzahl der verfügbaren Frequenzen $K$ hängt von der zeitlichen Auflösung der Messhardware und von der genutzten Messtrajektorie ab.

Zur Bestimmung der Systemfunktion gibt es verschiedene Verfahren, welche entweder auf einem Modell der MPI-Signalkette oder einer Kalibrierungsmessung basieren. Bei dem bisherigen Standardverfahren wird eine kleine mit Partikeln gefüllte Probe (Kalibrierprobe) in der Form eines Bildvoxels verwendet. Platziert man die Probe an einer Position r' und nimmt anschließend eine Messung $\hat{u}_k$' mit dem MPI-System auf, kann die Systemfunktion an dieser Stelle durch

$$s_k(r') \approx \frac{\hat{u}_k'}{V_0 c_0} \qquad (2)$$

approximiert werden. Dabei bezeichnet $V_0$ das Volumen und $c_0$ die Partikelkonzentration der Kalibrierprobe. Je kleiner die Kalibrierprobe, desto besser ist die Approximation. Um die Systemfunktion an allen Positionen im Messbereich aufzunehmen, wird die Kalibrierprobe mit Hilfe eines Positionierungsroboters bewegt. Dabei wird abwechselnd die Kalibrierprobe zur nächsten Position gefahren und eine Messung mit dem MPI-System aufgenommen. Üblicherweise wird ein kartesisches Gitter mit Ortspunkten $r_n$, $n=0,...,N-1$ verwendet. Dabei bezeichnet N die Gesamtanzahl an Abtastpunkten.

Nach der Diskretisierung des Gleichungssystems (1) an den Punkten $r_n$, $n=0,...,N-1$, entsteht ein lineares Gleichungssystem

$$\hat{u} = Sc, \qquad (3)$$

das zur Rekonstruktion von MPI-Messdaten zu lösen ist. Während der Kalibrierungsmessung wird die Systemmatrix spaltenweise gemessen. Die n-te Kalibrierungsmessung wird mit dem Faktor $1/ (V_0 c_0)$ multipliziert und in der n-ten Spalte der Systemmatrix eingetragen.

Das zuvor beschriebene Verfahren zur Bestimmung der Systemfunktion hat den Vorteil, dass es die tatsächlichen physikalischen Vorgänge während eines MPI-Experimentes bestimmen kann. Ein Nachteil des Verfahrens ist jedoch, dass die so bestimmte Systemfunktion in gewissen Teilen verrauscht sein kann, da eine MPI-Messung grundsätzlich Rauschen enthält. Ein noch wesentlicherer Nachteil ist jedoch der zeitliche Aufwand des Verfahrens. In [WGR09] benötigte die Bestimmung einer 3D Systemfunktion auf einem groben Gitter der Größe 34x20x28, welches ein Messfeld von 20.4 x 12 x 16.8 mm$^3$ abdeckt, ca. 6 Stunden. Auf einem feineren Gitter würde die Bestimmung der Systemfunktion Tage bis Monate benötigen, was in der Praxis nicht anwendbar ist.

Ein alternatives Verfahren wurde in [KSB10] vorgestellt. Dabei wird statt einer aufwändigen Kalibrierungsmessung ein Modell der MPI-Signalkette verwendet. Das Verfahren ist wesentlich schneller, besitzt jedoch den Nachteil, dass es nicht an die Genauigkeit der gemessenen Systemfunktion herankommt. Dies liegt vor allem daran, dass bislang kein Partikelmodell gefunden wurde, welches das physikalische Verhalten der Partikel ausreichend genau beschreibt.

Die Größe der Kalibrierprobe und die Größe eines Bildvoxels müssen nicht übereinstimmen. So macht es z.B. Sinn eine größere Kalibrierprobe zu verwenden, um das Signal-zu-Rausch-Verhältnis der Messdaten zu verbessern. In diesem Fall muss man jedoch mit einem Verlust der Auflösung rechnen.

In [HBRGB12] wurde ein Kalibrierverfahren vorgeschlagen, bei dem die mechanische Bewegung der Punktprobe durch eine elektromagnetische Bewegung der Magnetfelder ersetzt wird. Hierzu wird die Kalibrierprobe an einen statischen Ort positioniert. Anschließend wird sukzessive an dem Ort der Kalibrierprobe dasselbe statische Magnetfeld erzeugt, welches an den zu vermessenden Voxel-Positionen im Messfeld herrscht. Hierdurch lässt sich die Kalibriermessung beschleunigen, da eine elektromagnetische Feldänderung schneller realisierbar als eine mechanische Bewegung der Kalibrierprobe ist. Das in dieser Erfindung vorgeschlagene Verfahren ist davon unabhängig, ob die Kalibrierdaten durch mechanische oder elektromagnetische Schritte aufgenommen werden.

Während in [WGR09] das komplette Messvolumen durch die Messtrajektorie abgedeckt wird, wird bei größeren MPI-Scannern (z.B. für Anwendungen im Humanbereich) wohl nur ein kleiner Bereich des gesamten Messvolumens (= Patientenvolumen) durch Variation des Anregungsfelds abgedeckt werden können. In diesem Fall kann ein Multi-Station-Ansatz verwendet werden, bei dem kleine Subvolumina sukzessive abgefahren werden. Zu jedem Subvolumen muss in diesem Fall eine dedizierte Systemmatrix aufgenommen werden. Das in dieser Erfindung vorgeschlagene Verfahren lässt sich in diesem Fall auf jedes Subvolumen anwenden.

### Aufgabe der Erfindung

Aufgabe der vorliegenden Erfindung ist es demgegenüber, ein effizientes Verfahren zur Bestimmung der Systemmatrix für das bildgebende MPI-Verfahren anzugeben, welches eine MPI-Systemfunktion mit geringem zeitlichem Aufwand bestimmt und dabei trotzdem eine hohe Genauigkeit erreicht.

### Kurze Beschreibung der Erfindung

Erfindungsgemäß wird diese Aufgabe auf überraschend einfache, aber wirkungsvolle Weise dadurch gelöst, dass im Kalibrierverfahren Compressed-Sensing-Schritte mit einer Transformationsmatrix, die die Bildrekonstruktionsmatrix sparsifiziert, angewendet werden, wobei die Transformationsmatrix analytische Transformationen, eine modellierte MPI-Systemmatrix, eine simulierte oder eine gemessene Systemmatrix umfasst, und dass lediglich eine Anzahl $M < N$ Kalibrier-MPI-Messungen für M Voxel durchgeführt wird, wobei die M Voxel nach einem Zufallsverfahren oder einem Pseudo-Zufallsverfahren ausgewählt werden, und dass daraus die Bildrekonstruktionsmatrix erstellt und abgespeichert wird.

Das erfindungsgemäße Verfahren basiert auf der Erkenntnis, dass die MPI-Systemfunktion extrem gut komprimierbar ist. Wie in [Lam12] gezeigt wurde kann jede Zeile der MPI-Systemmatrix durch eine Basistransformation in der Form

$$s_k = \boldsymbol{B} v_k \qquad (4)$$

dargestellt werden. Dabei bezeichnet $\boldsymbol{s}_k$ die $k$-te Zeile der Systemmatrix S. Weiterhin beschreibt $\boldsymbol{v}_k$ den Koeffizientenvektor zur Darstellung der $k$-ten Zeile in der Basis $\boldsymbol{B}$. Bei geeigneter Wahl der Basistransformation enthält der Koeffizientenvektor nur sehr wenige Einträge ungleich Null. Der Großteil der Koeffizienten ist exakt Null oder hat einen verschwindend geringen Wert, so dass diese Koeffizienten in guter Approximation entfernt werden können. Eine Anwendung der Entwicklung einer Systemfunktion in Basisfunktionen besteht darin, die Systemfunktion zu komprimieren wodurch die Rekonstruktionszeit wesentlich verkürzt werden kann. Dazu wird die Systemfunktion zunächst hochaufgelöst bestimmt und anschließend in den dünn besetzten Raum der Basisfunktionen überführt [Lam12]. Das Problem der schnellen Bestimmung der Systemfunktion löst dieses Verfahren jedoch nicht.

Mit der vorliegenden Erfindung wird vorgeschlagen, ein in der Literatur als Compressed-Sensing (etwa aus Referenz [BT09] oder aus der Publikation Candes, E.J.; Ecole Polytech., Paris; Wakin, M.B.: "An Introduction To Compressive Sampling", Signal Processing Magazine, IEEE (Volume:25 , Issue: 2)) in anderem Zusammenhang an sich bekanntes Verfahren zu verwenden, um die Koeffizienten $\boldsymbol{v}_k$ schon mit nur wenigen MPI-Kafibrierungsmessungen zu bestimmen. Die Kalibrierungsmessungen dazu seien an $M << N$ Positionen $\boldsymbol{r}_m$, $m=0,...,M-1$ bestimmt und als Matrix mit Zeilenvektoren $\boldsymbol{y}_k$ abgespeichert worden. Mittels einer reduzierten Basistransformationsmatrix $\boldsymbol{B}_r$ kann $\boldsymbol{y}_k$ durch

$$y_k = \boldsymbol{B}_r \boldsymbol{v}_k \qquad\qquad (5)$$

dargestellt werden. Da dieses Gleichungssystem stark unterbestimmt ist, hat es unendlich viele Lösungen. Um dennoch den Koeffizientenvektor $\boldsymbol{v}_k$ bestimmen zu können machen sich Compressed-Sensing-Algorithmen zunutze, dass die zu bestimmende Lösung nur wenige von Null verschiedene Einträge hat. Neben der Sparsität der Lösung setzen diese Algorithmen voraus, dass die Transformationsmatrix $\boldsymbol{B}_r$ inkohärent ist. Dies kann erreicht werden, indem die Abtastpunkte $\boldsymbol{r}_m$, m=0,..., M-1 zufällig oder pseudozufällig gewählt werden.

Mit dem Vorwissen, dass die gesuchten Koeffizienten dünn besetzt sind, können diese mit dem folgenden Ansatz dennoch bestimmt werden:

$$\text{Minimiere } \|v_k\| \text{ unter der Nebenbedingung } y_k = \boldsymbol{B}_r \boldsymbol{v}_k. \qquad (6)$$

Hierbei kann die Norm unterschiedlich gewählt werden. Z.B können die Normen $\|v_k\|_0$ oder $\|v_k\|_1$ verwendet werden, wobei üblicherweise die |1-Norm verwendet wird. Da die Kalibrierungsmessungen $\boldsymbol{y}_m$ unter Umständen verrauscht sind, kann der Ansatz

$$\text{Minimiere } \|v_k\| \text{ unter der Nebenbedingung } \|y_k - \boldsymbol{B}_r \boldsymbol{v}_k\|_2 < \varepsilon \qquad (7)$$

zu besseren Ergebnissen führen. Dabei erlaubt man mit dem Parameter $\varepsilon$ eine gewisse Abweichung von den gemessenen Daten zu der berechneten Lösung. Zum allgemeinen Lösen der Probleme (6) und (7) wurden zahlreiche Algorithmen entwickelt. Einige der effizientesten Algorithmen sind der SL0-Algorithmus [HMC09] und der FISTA-Algorithmus [BT09].

Bevorzugte Ausführungsformen der Erfindung

Varianten sowie weitere vorteilhafte Eigenschaften und Ausgestaltungen der Erfindung sind im Folgenden beschrieben.

Eine einfache, an den bekannten und derzeit angewandten Verfahren aus dem Stand der Technik orientierte Klasse von Varianten des erfindungsgemäßen Verfahrens zeichnet sich dadurch aus, dass die Kalibrierprobe innerhalb des Untersuchungsvolumens an die Orte der M Voxel verschoben und jeweils eine Kalibrier-MPI-Messung ausgeführt wird. Diese Verfahrensvarianten sind mit einer Messmechanik, wie sie bei bekannten MPI-Apparaturen üblicherweise vorhanden ist, ohne Weiteres durchführbar.

Alternativ kann bei einer weiteren Klasse von Varianten des erfindungsgemäßen Verfahrens während der Kalibrier-MPI-Messungen die Kalibrierprobe stationär bleiben und Magnetfelder der MPI-Apparatur derart variiert werden, dass die statischen Magnetfelder, die an den Orten der M Voxel herrschen, nacheinander nachgebildet werden. Damit lässt sich - insbesondere bei neu herzustellenden MPI-Apparaturen - die relativ aufwändige Verschiebemechanik einsparen.

Eine dritte Klasse von Varianten des erfindungsgemäßen Verfahrens ist dadurch gekennzeichnet, dass während der Kalibrier-MPI-Messungen die Kalibrierprobe stationär bleibt und dass Magnetfelder einer von der MPI-Apparatur unabhängigen System-Kalibriereinheit derart variiert werden, dass die statischen Magnetfelder, die an den Orten der M Voxel herrschen, nacheinander nachgebildet werden. Eine dedizierte System-Kalibriereinheit kann das Signal-zu-Rausch-Verhältnis der Kalibriermessung gegenüber einer "normalen" MPI-Apparatur deutlich verbessern.

Bevorzugt ist eine Variante des erfindungsgemäßen Kalibrierverfahren, bei welcher das Volumen der Kalibrierprobe größer ist als das Volumen eines Voxels, wodurch ein Gewinn an Signalintensität und dadurch ein besseres Signal-zu-Rausch-Verhältnis der Kalibriermessung erreicht wird, allerdings im Allgemeinen auf Kosten der Auflösung.

Um das Signal-zu-Rausch-Verhältnis der Kalibriermessung zu verbessern, ohne dabei die Auflösung beim erfindungsgemäßen Verfahrens zu verschlechtern, kann bei einer Weiterbildung der obigen Verfahrensvariante vorgesehen sein, dass die Kalibrierprobe eine nicht-homogene innere Struktur aufweist. So ist es beispielsweise denkbar, die Kalibrierprobe aus einer Anzahl von Voxeln mit Zwischenräumen aufzubauen, wobei die Zwischenräume zur Stabilisierung der Kalibrierprobe nichtmagnetisches Material enthalten können. Denkbar sind auch Kalibrierproben, die eine nicht-homogene Struktur besitzen und verschiedene Voxel unregelmäßig abdecken.

Das mit der vorliegenden Erfindung vorgestellte Verfahren hängt entscheidend davon ab, dass die Basistransformation B geeignet gewählt wird. Im Folgenden sollen hierfür unterschiedliche Möglichkeiten angeboten werden:

- Analytische Transformationen, wie z.B. die diskrete FourierTransformation (DFT), die diskrete Cosinus-Transformation (DCT), die diskrete Wavelet-Transformation (DWT), oder die diskrete Chebyshev-Transformation (DTT). Im Falle der mehrdimensionalen Bildgebung (2D / 3D) können die mehrdimensionalen Varianten der zuvor genannten Transformationen genutzt werden.

- Eine modellierte MPI-Systemmatrix. Da eine modellierte MPI-Systemmatrix bereits eine deutlich größere Ähnlichkeit mit der echten Systemmatrix als die oben genannten analytischen Transformationsmatrizen hat, kann die Sparsität erhöht und damit die Anzahl der benötigten Kalibriermessungen verringert werden.

- Eine gemessene MPI-Systemmatrix. Das Szenario dabei wäre folgendes: Es wird eine hochaufgelöste Systemmatrix gemessen. Wenn sich zu einem späteren Zeitpunkt die Messparameter oder die Partikelcharge ändern, kann schon mit wenigen Kalibrierungsmessungen eine neue hochaufgelöste Systemmatrix bestimmt werden. Hierbei wird ausgenutzt, dass sich die Systemmatrix bei einer Änderung der Messparameter oder der Partikelcharge nur geringfügig ändert.

Wenn die Koeffizienten $v_k$ bestimmt worden sind, kann die volle Systemmatrix durch Auswerten von (4) bestimmt werden.

Parameterwahl

Der Gewinn des in dieser Erfindung vorgeschlagenen Verfahrens hängt erheblich von der Wahl der Parameter N und M ab. Für kleine N (<100) lohnt sich der Aufwand einer Compressed-Sensing-Rekonstruktion der Systemmatrix aller Voraussicht nach nicht. Für größere N sollte M gerade so gewählt werden, dass mit einer möglichst geringen Anzahl, ein gutes Ergebnis erzielt werden kann. Bei den im Anwendungsbeispiel genutzten 2D-Daten konnte bei einer Reduzierung auf 10% der Kalibriermessungen ein sehr gutes Ergebnis erzielt werden. Bei anderen Transformationsmatrizen (simulierte / gemessene Systemmatrix) lassen sich aller Voraussicht nach die Anzahl der erforderlichen Kalibrierungsmessungen noch deutlich reduzieren. Im Falle von 3D-Messungen kann zudem das Verhältnis von M zu N noch wesentlich geringer als bei 2D-Messungen gewählt werden.

Bevorzugt werden beim erfindungsgemäßen Kalibrierverfahren M und N daher so gewählt, dass gilt:

$$100 \leq N < 10^{12}$$

und

M/N $\leq$ 0,5, vorzugsweise M/N < 0,1, insbesondere M/N < $10^{-3}$.

Für die Bildrekonstruktion muss die MPI-Bildgebungsgleichung mit einem geeigneten Verfahren gelöst werden. Während hierzu üblicherweise die Bildgebungsmatrix im Frequenzbereich betrachtet wird, wurde in [GC11] vorgeschlagen, die Matrix im Zeitbereich zu betrachten. Hierdurch kann unter der Annahme von idealisierten Magnetfeldern, die MPI-Bildgebungsgleichung als Faltung geschrieben werden. Das in [GC11] beschriebene "X-Space"-Rekonstruktionsverfahren kann durch direktes Gridding ein Bild relativ geringer Auflösung liefern ("native resolution") oder durch Gridding mit nachfolgender Entfaltung ein Bild mit höherer Auflösung erzeugen. Zudem existieren für die direkte Lösung einer Faltungsgleichung sehr effiziente Algorithmen, welche dann für die MPI-Bildrekonstruktion genutzt werden können. Für den Entfaltungsansatz oder das direkte Lösen der Faltungsgleichung benötigt man einen Faltungskern.

Das in dieser Erfindung vorgeschlagene Verfahren lässt sich auch dazu nutzen, diesen Faltungskern zu bestimmen. Hierzu kann der Faltungskern mit dem beschriebenen Compressed-Sensing-Ansatz direkt im Zeitbereich bestimmt werden. Um eine Ortsabhängigkeit des Faltungskerns zu erfassen, kann der Kern zu unterschiedlichen Zeitpunkten der Trajektorie bestimmt werden. Die Information über den Faltungskern steckt auch in der frequenz-basierten Systemfunktion. Um den Faltungskern zu bestimmen, muss die im Frequenzbereich bestimmte Systemmatrix in den Zeitbereich überführt werden. Anschließend repräsentieren die Matrixzeilen den Faltungskern, welcher in Abhängigkeit von der Zeilennummer im Ort verschoben ist und unter Umständen auch ortsabhängig sein kann.

Besonders bevorzugt ist daher eine Variante des erfindungsgemäßen Kalibrierverfahrens, die sich dadurch auszeichnet, dass aus der erstellten und abgespeicherten Bildrekonstruktionsmatrix ein - gegebenenfalls ortsabhängiger - Faltungskern bestimmt und damit bei der anschließenden Durchführung einer X-Space Rekonstruktion die räumliche Auflösung verbessert wird.

In den Rahmen der vorliegenden Erfindung fallen auch MPI-Abbildungsverfahren, die jeweils die Durchführung eines MPI-Experiments beinhalten und eine Bildrekonstruktionsmatrix verwenden, die nach dem Kalibrierverfahren gemäß der oben beschrieben Art erstellt wird.

Außerdem fällt in den Rahmen der Erfindung auch eine MPI-Apparatur zur Durchführung des Kalibrierverfahrens und/oder des MPI-Abbildungsverfahrens der oben beschriebenen Art, welche sich dadurch auszeichnet, dass eine elektronische Steuereinheit vorgesehen ist, die eine Transformationsmatrix zur Sparsifizierung der Bildrekonstruktionsmatrix gespeichert hat und mit dieser im Kalibrierverfahren Compressed-Sensing-Schritte anwenden kann, wobei die Steuereinheit eine Anzahl M < N Kalibrier-MPI-Messungen für M Voxel durchführen und daraus die Bildrekonstruktionsmatrix erstellen und abspeichern kann.

Zeichnung und detaillierte Beschreibung eines Ausführungsbeispiels

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die Daten des gezeigten und beschriebenen Ausführungsbeispiels sind nicht als abschließende Aufzählung zu verstehen, sondern haben vielmehr beispielhaften Charakter für die Schilderung der Erfindung.

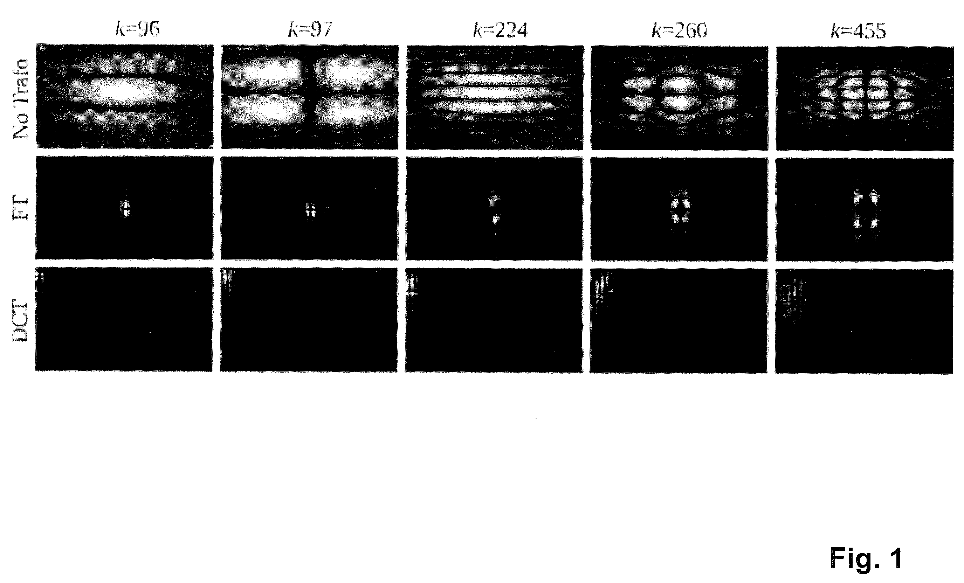

Fig. 1     zeigt fünf ausgewählte Zeilen der MPI-Systemmatrix vor und nach Anwenden einer Basistransformation (4). Als Transformationen wurden die DFT und die DCT verwendet. Wie man sieht, sind die transformierten MPI-Systemmatrixzeilen nur an wenigen Stellen ungleich Null.

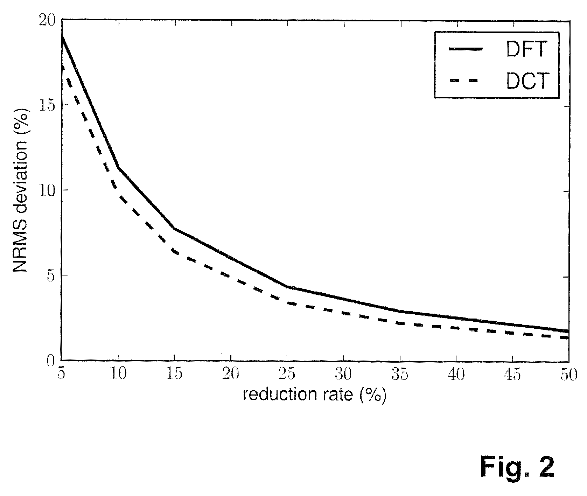

Fig. 2     zeigt den relativen Fehler der mittels des vorgeschlagenen Verfahrens bestimmten Systemmatrizen im Vergleich zu einer voll abgetasteten Systemmatrix wobei die Anzahl der Kalibrierungsmessungen zwischen 5% und 50% der vollen Anzahl an Abtastpunkten liegt (N=2720). Bis zu einer Reduktion auf 10% liegt der Fehler unter 10% und somit im tolerierbaren Bereich.

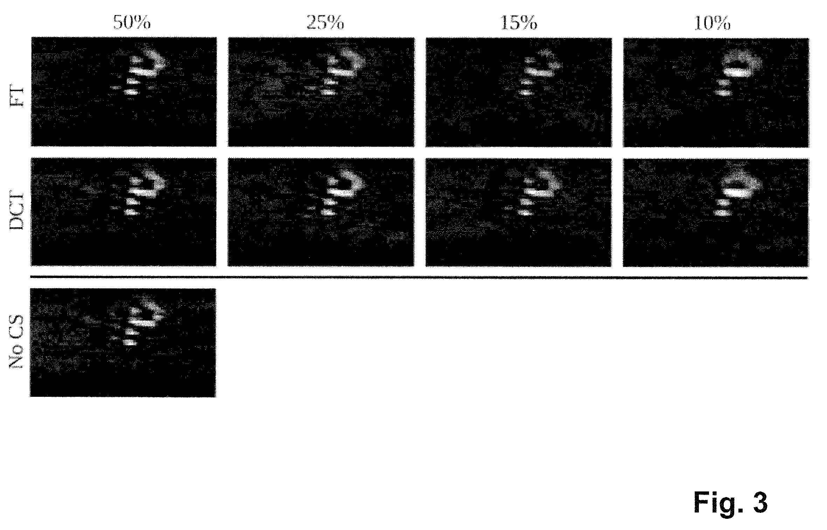

Fig. 3     zeigt rekonstruierte MPI-Daten, die mit den durch Compressed-Sensing rekonstruierten Systemmatrizen bestimmt wurde. Als Referenz dient ein mit einer voll abgetasteten Systemmatrix rekonstruierter Datensatz. Bis zu einer Reduktion auf 15% ist kein Qualitätsverlust in den rekonstruierten Daten erkennbar.

Parameter der Beispieldaten des in den Figuren dargestellten Anwendungsbeispiels:

Zum Evaluieren der Erfindung wurden 2D-MPI-Daten aufgenommen, die ein mit Partikeln gefülltes Phantom abbilden. Das Phantom besteht aus 12 Punkten, die den Buchstaben P darstellen. Als Compressed-Sensing Verfahren wurde der FISTA-Algorithmus [BT09] genutzt und der Parameter $\varepsilon$ so gewählt, dass die Ergebnisse optimal sind. Die Anzahl der Bildpunkte liegt bei 68x40 (N=2720). Das Messfeld deckt einen Bereich von 20.4 mm x 12 mm ab. Die Kalibrierprobe besteht aus einem Würfel mit Kantenlänge 0.6 mm und ist wie das Phantom mit dem Tracer Resovist gefüllt.

Referenzen

[WGR09] J. Weizenecker, B. Gleich, J. Rahmer, H. Dahnke, and J. Borgert. Three- dimensional real-time in vivo magnetic particle imaging. Phys. Med. Biol., 54(5):L1-L10, 2009.

[KSB10] T. Knopp, T. F. Sattel, S. Biederer, J. Rahmer, J. Weizenecker, B. Gleich, J. Borgert, and T. M. Buzug. Model-based reconstruction for magnetic particle imaging. IEEE Trans. Med. Imaging, 29(1):12-18, 2010.

[LDP07] M. Lustig, D. Donoho, J.M. Pauly. Sparse MRI: The application of compressed sensing for rapid MR imaging. Magn. Reson. Med. 2007 Dec; 58(6):1182-95.

[Lam12] J Lampe et al. Fast reconstruction in magnetic particle imaging. Phys. Med. Biol. 2012, 57.

[HMC09] Hossein Mohimani, Massoud Babaie-Zadeh, Christian Jutten, "A fast approach for overcomplete sparse decomposition based on smoothed L0 norm", IEEE Transactions on Signal Processing, Vol.57, No.1, January 2009, pp. 289-301.

[BT09] Amir Beck, Marc Teboulle A Fast Iterative Shrinkage-Thresholding Algorithm for Linear Inverse Problems, SIAM Journal on Imaging Sciences (2009) Volume: 2, Issue: 1, Pages: 183

[HBRGB12] A. Halkola, T. Buzug, J. Rahmer, B. Gleich, and C. Bontus, "System calibration unit for magnetic particle imaging: Focus field based system function," in Springer Proceedings in Physics Volume 140, vol. 42, Lübeck, March 2012, pp. 27-31.

[GC11] P. W. Goodwill and S. M. Conolly, Multidimensional X-Space Magnetic Particle Imaging, IEEE Trans. Med. Imag., 30(9), 1581-1590, 2011.

**Patentansprüche**

1. Kalibrierverfahren einer MPI-Apparatur zur Durchführung eines MPI-Experiments, wobei das Kalibrierverfahren M Kalibrier-MPI-Messungen mit einer Kalibrierprobe umfasst und aus diesen eine Bildrekonstruktionsmatrix ($y_k$) erzeugt, mit der die Signalbeiträge von N Voxeln innerhalb eines Untersuchungsvolumens der MPI-Apparatur ermittelt werden,
   **dadurch gekennzeichnet,**
   **dass** im Kalibrierverfahren Compressed-Sensing-Schritte mit einer Transformationsmatrix ($B_r$), die die Bildrekonstruktionsmatrix ($y_k$) sparsifiziert, angewendet werden,
   wobei die Transformationsmatrix ($B_r$) analytische Transformationen, eine modellierte MPI-Systemmatrix, eine simulierte oder eine gemessene Systemmatrix umfasst,
   und **dass** lediglich eine Anzahl M < N Kalibrier-MPI-Messungen für M Voxel durchgeführt wird, wobei die M Voxel nach einem Zufallsverfahren oder einem Pseudo-Zufallsverfahren ausgewählt werden, und dass daraus die Bildrekonstruktionsmatrix ($y_k$) erstellt und abgespeichert wird.

2. Kalibrierverfahren nach Anspruch 1, **dadurch gekennzeichnet, dass** die Kalibrierprobe innerhalb des Untersuchungsvolumens an die Orte der M Voxel verschoben und jeweils eine Kalibrier-MPI-Messung ausgeführt wird.

3. Kalibrierverfahren nach Anspruch 1, **dadurch gekennzeichnet, dass** während der Kalibrier-MPI-Messungen die Kalibrierprobe stationär bleibt und Magnetfelder der MPI-Apparatur derart variiert werden, dass die statischen Magnetfelder, die an den Orten der M Voxel herrschen, nacheinander nachgebildet werden.

4. Kalibrierverfahren nach Anspruch 1, **dadurch gekennzeichnet, dass** während der Kalibrier-MPI-Messungen die Kalibrierprobe stationär bleibt und Magnetfelder einer von der MPI-Apparatur unabhängigen System-Kalibriereinheit derart variiert werden, dass die statischen Magnetfelder, die an den Orten der M Voxel herrschen, nacheinander nachgebildet werden.

5. Kalibrierverfahren nach einem der vorhergehenden Ansprüche, **dadurch gekennzeichnet, dass** das Volumen der Kalibrierprobe größer ist als das Volumen eines Voxels.

6. Kalibrierverfahren nach Anspruch 5, **dadurch gekennzeichnet, dass** die Kalibrierprobe eine nicht-homogene innere Struktur aufweist.

7. Kalibrierverfahren nach einem der Ansprüche 1 bis 6, **dadurch gekennzeichnet, dass** die Transformationsmatrix ($B_r$) die diskrete FourierTransformation (=DFT), die diskrete Cosinus-Transformation (=DCT), die diskrete Wavelet-Transformation (=DWT) oder die diskrete Chebyshev-Transformation (=DTT) umfasst, wobei im Falle der mehrdimensionalen Bildgebung (2D / 3D) die mehrdimensionalen Varianten der genannten Transformationen genutzt werden können.

8. Kalibrierverfahren nach einem der vorhergehenden Ansprüche, **dadurch gekennzeichnet, dass** gilt:

$$100 < N < 10^{12}$$

und M/N < 0,5, vorzugsweise M/N < 0,1, insbesondere M/N < $10^{-3}$.

9. Kalibrierverfahren nach einem der vorhergehenden Ansprüche, **dadurch gekennzeichnet, dass** aus der erstellten und abgespeicherten Bildrekonstruktionsmatrix ($y_k$) ein - gegebenenfalls ortsabhängiger - Faltungskern bestimmt und damit bei der anschließenden Durchführung einer X-Space Rekonstruktion die räumliche Auflösung verbessert wird.

**10.** MPI-Abbildungsverfahren, das die Durchführung eines MPI-Experiments beinhaltet und eine Bildrekonstruktions-matrix ($y_k$) verwendet, die nach dem Kalibrierverfahren gemäß einem der vorhergehenden Ansprüche erstellt wird.

**11.** MPI-Apparatur geeignet zur Durchführung des Kalibrierverfahrens nach einem der Ansprüche 1-9 und/oder des MPI-Abbildungsverfahrens nach Anspruch 10, **dadurch gekennzeichnet, dass** eine elektronische Steuereinheit vorgesehen ist, die sämtliche Verfahrensschritte nach Anspruch 1 ausführt und eine Transformationsmatrix ($B_r$) zur Sparsifizierung der Bildrekonstruktionsmatrix ($y_k$) gespeichert hat und mit dieser im Kalibrierverfahren Compressed-Sensing-Schritte anwenden kann, und dass die Steuereinheit eine Anzahl M < N Kalibrier-MPI-Messungen für M Voxel durchführt und daraus die Bildrekonstruktionsmatrix ($y_k$) erstellt und abspeichert.

**Claims**

**1.** Calibration method of an MPI apparatus for performing an MPI experiment, wherein the calibration method comprises M calibration MPI measurements with a calibration sample and uses these measurements to create an image reconstruction matrix ($y_k$) with which the signal contributions of N voxels within a volume under investigation of the MPI apparatus are determined,
**characterized in that**
compressed sensing steps are applied in the calibration method with a transformation matrix ($B_r$) that sparsifies the image reconstruction matrix ($y_k$),
wherein the transformation matrix ($B_r$) comprises analytic transformations, a modelled MPI-system matrix, a simulated or a measured system matrix
and **in that** only a number of M<N calibration MPI measurements for M voxels are carried out, wherein the M voxels are selected in a random or pseudo-random fashion and are used for creating and storing the image reconstruction matrix ($y_k$).

**2.** Calibration method according to claim 1, **characterized in that** the calibration sample is shifted within the volume under investigation to the locations of the M voxels and one calibration MPI measurement is performed for each voxel.

**3.** Calibration method according to claim 1, **characterized in that** the calibration sample remains stationary during the calibration MPI measurements and magnetic fields of the MPI apparatus are varied in such a fashion that the static magnetic fields that prevail at the locations of the M voxels are reconstructed, one after another.

**4.** Calibration method according to claim 1, **characterized in that** the calibration sample remains stationary during the calibration MPI measurements and magnetic fields of a system calibration unit that is independent of the MPI apparatus are varied in such a fashion that the static magnetic fields that prevail at the locations of M voxels are reproduced, one after another.

**5.** Calibration method according to any one of the preceding claims, **characterized in that** the volume of the calibration sample is larger than the volume of a voxel.

**6.** Calibration method according to claim 5, **characterized in that** the calibration sample has a non-homogeneous inner structure.

**7.** Calibration method according to any one of the claims 1 through 6, **characterized in that** the transformation matrix ($B_r$) includes Discrete Fourier Transformation (=DFT), Discrete Cosine Transformation (=DCT), Discrete Wavelet Transformation (=DWT), or discrete Chebyshev Transformation (=DTT), wherein in case of multi-dimensional imaging (2D / 3D), the multi-dimensional variants of the mentioned transformations can be utilized.

**8.** Calibration method according to any one of the preceding claims, **characterized in that** the following applies:

$$100 < N < 10^{12}$$

and M/N < 0.5, advantageously M/N < 0.1, in particular M/N < $10^{-3}$.

**9.** Calibration method according to any one of the preceding claims, **characterized in that** a convolution kernel - which can be spatially dependent if appropriate - is determined from the created and stored image reconstruction matrix ($y_k$) and used for improving the spatial resolution in subsequent performance of an X space reconstruction.

10. MPI imaging method including the performance of an MPI experiment and using an image reconstruction matrix ($y_k$) that is created using the calibration method in accordance with any one of the preceding claims.

11. MPI apparatus which is suitable for performing the calibration method according to any one of the claims 1 to 9 and/or the MPI imaging method according to claim 10, **characterized in that** an electronic control unit is provided which performs all steps according to claim 1 and contains a stored transformation matrix ($B_r$) for sparsifying the image reconstruction matrix ($y_k$) which enables application of compressed-sensing steps in the calibration method, and that the control unit performs a number of M<N calibration-MPI measurements for M voxels from which the image reconstruction matrix ($y_k$) is generated and stored.

## Revendications

1. Procédé d'étalonnage d'un appareil d'imagerie à particules magnétiques (MPI) pour exécuter une expérience de MPI, le procédé d'étalonnage comprenant M mesures de MPI d'étalonnage avec un échantillon d'étalonnage et produisant à partir de celles-ci une matrice de reconstruction d'image ($y_k$) au moyen de laquelle les contributions au signal de N voxels à l'intérieur d'un volume d'analyse de l'appareil de MPI sont déterminées,
**caractérisé en ce**
**que** des étapes d'acquisition comprimée sont appliquées lors du procédé d'étalonnage au moyen d'une matrice de transformation ($B_r$) qui rend parcimonieuse la matrice de reconstruction d'image ($y_k$),
la matrice de transformation ($B_r$) comprenant des transformations analytiques, une matrice du système MPI modélisée, une matrice du système simulée ou mesurée, que seul un nombre M < N de mesures de MPI d'étalonnage pour M voxels est exécuté, les M voxels étant sélectionnés selon un procédé, aléatoire ou un procédé pseudo-aléatoire,
et **que** la matrice de reconstruction d'image ($y_k$) est créée et enregistrée à partir de ces mesures.

2. Procédé d'étalonnage selon la revendication 1, **caractérisé en ce que** l'échantillon d'étalonnage est déplacé à l'intérieur du volume d'analyse aux lieux des M voxels et une mesure de MPI d'étalonnage est exécutée à chaque fois.

3. Procédé d'étalonnage selon la revendication 1, **caractérisé en ce que** pendant les mesures de MPI d'étalonnage, l'échantillon d'étalonnage reste stationnaire et on fait varier les champs magnétiques de l'appareil de MPI de telle façon que les champs magnétiques statiques qui règnent aux lieux des M voxels soient successivement reproduits.

4. Procédé d'étalonnage selon la revendication 1, **caractérisé en ce que** pendant les mesures de MPI d'étalonnage, l'échantillon d'étalonnage reste stationnaire et on fait varier les champs magnétiques d'une unité d'étalonnage du système indépendante de l'appareil de MPI de telle façon que les champs magnétiques statiques qui règnent aux lieux des M voxels soient successivement reproduits.

5. Procédé d'étalonnage selon l'une des revendications précédentes, **caractérisé en ce que** le volume de l'échantillon d'étalonnage est plus grand que le volume d'un voxel.

6. Procédé d'étalonnage selon la revendication 5, **caractérisé en ce que** l'échantillon d'étalonnage présente une structure interne non homogène.

7. Procédé d'étalonnage selon l'une des revendications 1 à 6, **caractérisé en ce que** la matrice de transformation ($B_r$) comprend la transformation de Fourier discrète (= TFD), la transformation en cosinus discrète (= TCD), la transformation en ondelettes discrète (= TOD) ou la transformation de Tchebychev discrète (= DTT), les variantes multidimensionnelles des transformations mentionnées pouvant être utilisées dans le cas de l'imagerie multidimensionnelle (2D / 3D).

8. Procédé d'étalonnage selon l'une des revendications précédentes, **caractérisé en ce que** :

$$100 < N < 10^{12}$$

et M/N < 0,5, de préférence M/N < 0,1, en particulier M/N < $10^{-3}$.

9. Procédé d'étalonnage selon l'une des revendications précédentes, **caractérisé en ce qu'**un noyau de convolution - le cas échéant dépendant du lieu - est déterminé à partir de la matrice de reconstruction d'image ($y_k$) créée et enregistrée et par conséquent la résolution spatiale améliorée lors de l'exécution suivante d'une reconstruction dans l'espace X.

10. Procédé d'imagerie par MPI qui comprend l'exécution d'une expérience de MPI et utilise une matrice de reconstruction d'image ($y_k$) qui est créée d'après le procédé d'étalonnage selon l'une des revendications précédentes.

11. Appareil de MPI conçu pour exécuter le procédé d'étalonnage selon l'une des revendications 1 à 9 et/ou le procédé d'imagerie par MPI selon la revendication 10, **caractérisé en ce qu'**il est prévu une unité de commande électronique qui exécute toutes les étapes du procédé selon la revendication 1 et dans laquelle est enregistrée une matrice de transformation ($B_r$) pour rendre parcimonieuse la matrice de reconstruction d'image ($y_k$) et qui peut appliquer avec celle-ci des étapes d'acquisition comprimée lors du procédé d'étalonnage, et que l'unité de commande exécute un nombre M < N de mesures de MPI d'étalonnage pour M voxels et crée et enregistre la matrice de reconstruction d'image ($y_k$) à partir de ces mesures.

| | $k=96$ | $k=97$ | $k=224$ | $k=260$ | $k=455$ |
|---|---|---|---|---|---|
| No Trafo | | | | | |
| FT | | | | | |
| DCT | | | | | |

**Fig. 1**

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- An Introduction To Compressive Sampling. **WAKIN, M.B.** Signal Processing Magazine. IEEE, vol. 25
- **J. WEIZENECKER ; B. GLEICH ; J. RAHMER ; H. DAHNKE ; J. BORGERT.** Three- dimensional real-time in vivo magnetic particle imaging. *Phys. Med. Biol.,* 2009, vol. 54 (5), L1-L10
- **T. KNOPP ; T. F. SATTEL ; S. BIEDERER ; J. RAHMER ; J. WEIZENECKER ; B. GLEICH ; J. BORGERT ; T. M. BUZUG.** Model-based reconstruction for magnetic particle imaging. *IEEE Trans. Med. Imaging,* 2010, vol. 29 (1), 12-18
- **M. LUSTIG ; D. DONOHO ; J.M. PAULY.** Sparse MRI: The application of compressed sensing for rapid MR imaging. *Magn. Reson. Med.,* Dezember 2007, vol. 58 (6), 1182-95
- **J LAMPE et al.** Fast reconstruction in magnetic particle imaging. *Phys. Med. Biol.,* 2012, 57
- **HOSSEIN MOHIMANI ; MASSOUD BABAIE-ZADEH ; CHRISTIAN JUTTEN.** A fast approach for overcomplete sparse decomposition based on smoothed L0 norm. *IEEE Transactions on Signal Processing,* Januar 2009, vol. 57 (1), 289-301
- **AMIR BECK ; MARC TEBOULLE.** A Fast Iterative Shrinkage-Thresholding Algorithm for Linear Inverse Problems. *SIAM Journal on Imaging Sciences,* 2009, vol. 2 (1), 183
- **A. HALKOLA ; T. BUZUG ; J. RAHMER ; B. GLEICH ; C. BONTUS.** System calibration unit for magnetic particle imaging: Focus field based system function. *Springer Proceedings in Physics,* Marz 2012, vol. 140, 27-31
- **P. W. GOODWILL ; S. M. CONOLLY.** Multidimensional X-Space Magnetic Particle Imaging. *IEEE Trans. Med. Imag.,* 2011, vol. 30 (9), 1581-1590